# EUROPEAN PATENT SPECIFICATION

(11) **EP 1 781 181 B1**
(45) Date of publication and mention of the grant of the patent: **15.07.2009**
(21) Application number: 05756362.9
(22) Date of filing: 25.05.2005
(51) Int. Cl.: A61B 17/068

(54) **APPLICATOR FOR DEPLOYING A SURGICAL FASTENER**
APPLIKATOR ZUR ABLAGE EINES CHIRURGISCHEN BEFESTIGUNGSELEMENTS
APPLICATEUR PERMETTANT DE DEPLOYER UNE AGRAFE CHIRURGICALE

(30) Priority: 25.06.2004 US 877669
(43) Date of publication of application: 09.05.2007
(73) Proprietor: ETHICON, INC., Somerville, New Jersey 08876-0151 (US)
(72) Inventor: KAMMERER, Gene W., East Brunswick, New Jersey 08816 (US); HOWANSKY, Mark, Union City, New Jersey 07087 (US); KNODEL, Bryan D., Flagstaff, Arizona 86001 (US)
(74) Representative: Fisher, Adrian John
(86) International application number: PCT/US2005/018376
(87) International publication number: WO 2006/007189

(56) References cited:
- WO-A-01/43625
- US-A- 5 807 403
- US-A- 5 941 439

## Description

### Field Of The Invention

This invention relates to applicators for deploying surgical fasteners in tissue. More particularly, it relates to certain applicators especially adapted for deploying surgical fasteners to attach tissues to each other, and to attach a mesh to tissue within a deep cavity of the body, such as the pelvic cavity.

### Background Of The Invention

In many situations, one piece of tissue must be attached to another piece of tissue for wound closure or the surgical repair of tissue defects. For example, an open wound or surgical incision may need to be closed following a surgical procedure. An injury may cause one piece of tissue (e.g., a tendon or pelvic floor tissue after childbirth) to become detached from another piece of tissue (e.g., a bone or pelvic muscle), or a piece of tissue may simply tear (e.g., a piece of meniscal cartilage or pelvic floor tissue).

The traditional technique for attaching one piece of soft tissue to another piece of soft tissue has involved stitching the two pieces of tissue together using suture. However, in many circumstances such stitching can be challenging either because of the time required to do the stitching or the difficulty of stitching in a particular area of the body. Other techniques have involved using both suture and mesh to support the tissue that has been torn or become degenerated.

More recently, different types of surgical fasteners have been developed for holding together two pieces of tissue. Among the fasteners which have been developed to date are the so-called T-type fasteners, in which a rod-like head is perpendicularly mounted to the end of a length of flexible filament. Another of these fasteners is the so-called H-type fastener, in which a rod-like head is perpendicularly mounted to the two opposite ends of an intermediate, bridging flexible filament. Appropriate applicator tools have also been developed for deploying such fasteners in tissue. Examples of T-type and H-type fasteners, and their associated applicators, are disclosed in U.S. Patents Nos. 4,006,747 (Kronenthal et al.); 4,235,238 (Ogiu et al.); 4,669,474 (Richards et al.); 4,705,040 (Mueller et al.); and 5,941,439 (Kammerer et al.). US 5 807 403 discloses an applicator comprising parallel hollow barrels for deploying a pair of suture attached anchors into bone.

In the case of pelvic floor repair, for example where a pelvic organ prolapses into the vagina, a prominent method of repairing these defect conditions is by a reduction of the prolapse sack through surgery. In certain cases the sack is cut down and the repair is made through suture closures. In other cases a supporting material is placed between the organ which is prolapsing into the vagina. These materials can be cadaver fascia, autologus fascia, animal derived grafts, or synthetic materials such as PROLENE® meshes or other polymeric fabrics or meshes. In the attachment method of these materials within the pelvic cavity, sutures and hand held needles are generally used. The needle is passed, one step at a time through the tissue, then through the material, and then through the tissue again. A deep cavity knot is then tied with multiple throws.

Another method which has been disclosed for supporting and repairing pelvic structures involves the insertion of trocars through the pelvic muscles to provide a passageway to the repair site. Suture or mesh can be attached to the trocars and consequently passed into the muscle structure. With respect to the mesh, friction between the mesh and surrounding tissue hold the mesh in place to support and repair the pelvic tissue. With respect to suture, the suture is usually required to be tied subcuticularlly. In either case, the insertion of trocars through the pelvic muscles is still very skill dependent.

Still another method to attach meshes or fabrics to the inside of the pelvic cavity is by placing bone anchors. In this case the anchors are set into the sacrum or pubic bone or ischial spine. A suture is passed through the anchor and the fabric is attached to the suture via knotting. Bone anchors can be painful to the patient as they are inserted through the periostium. Also, if they are metal, and if dislodged, they can migrate within the pelvic cavity. They are sometimes associated with infections of the bone if careful aseptic techniques are not followed since they do break the sealing tissue around the bone and penetrate into the bone.

In addition to these surgical interventions, there are some pelvic floor repair cases where a tissue shrinkage technique can help. In tissues that have a high content of collagen such as fascia, using an energy source to heat and hence shrink the collagen can effectively tighten the pelvic floor. However, this is a limited application and is generally reserved for procedures, which do not involve significant stretching of the vaginal tissue or breakage of the endopelvic fascia.

Unfortunately, none of these procedures to repair the pelvic floor by direct suturing or placement of a supportive mesh or bone anchor is optimal. Suturing by itself is time-consuming and skill dependent. The surgical procedures in which mesh is attached to the tissue or a bone anchor is used for attachment are also very skill dependent. This can lead to long operations in a surgical suite with general anesthesia followed by two to three day hospital stays and weeks of recovery. It can also lead to unpredictable outcomes. The energy based tissue shrinkage approach is typically limited to tissue close to or surrounding the urethra, and therefore has limited application. Accordingly, what is needed within the surgical community is an applicator especially adapted for deploying a surgical fastener to attach tissues to each other, and to attach a mesh to tissue within a deep cavity of the body, such as the pelvic cavity.

### Summary Of The Invention

The applicator of the invention is defined in appended claim 1. Further preferred features thereof are defined in subclaims 2-6.

The features of the present invention will be more fully disclosed by the following detailed description of the preferred embodiments of the invention, which is to be considered together with the accompanying drawings wherein like numbers refer to like parts.

### Brief Description Of The Drawings

Fig. 1 is a side view of an applicator formed in accordance with the present invention, with the applicator being shown in a first operating position;
Fig. 2 is a side view of the applicator of Fig. 1 , with the applicator being shown in a second operating position;
Figs. 3-7 are views showing construction details of selected portions of the applicator's push rod assembly;
Fig. 8 is a view showing construction details of selected portions of the applicator's guide shaft assembly; Figs. 9-11 are views showing construction details of selected portions of the applicator's needle assembly;
Fig. 12 is a perspective view showing one type of fastener which can be used in conjunction with the present invention;
Fig. 13 is a view of the fastener of Fig. 12 as it is held in the distal end of the applicator;
Fig. 14 is an assembly view of an alternate embodiment of an applicator formed in accordance with the present invention;
Fig. 15 is a side view of the applicator of Fig. 14 in a first operating position; Fig. 15A is side view of the applicator of Fig. 14 in a second operating position;
Fig. 15A is side view of the applicator of Fig. 14 in a second operating position;
Fig. 15B is a view showing construction details of selected portions of the push rod assembly of the applicator of Fig. 14;
Figs. 15C, 16 and 16A are views showing construction details of selected portions of the needle assembly of applicator of Fig. 14;
Figs 16B-16C are views of a shield assembly of the applicator of Fig. 14 in deployed and retracted positions respectively;
Figs 16D-16E are views of an alternate embodiment of a shield assembly of the applicator of Fig. 14 in deployed and retracted positions respectively;
Fig. 17 is yet another alternate embodiment of an applicator formed in accordance with the present invention, Fig. 18 is a view of the applicator of Fig. 17 with the applicator being shown in a first operating position;
Fig. 19 is a view of the connection of the guide shaft section to the housing of the applicator of Fig. 17;
Fig. 20 is a view of the applicator of Fig. 17 with the applicator being shown in a second operating position;
Fig. 21 is a view of the inside diameter of the trigger of the applicator of Fig. 17;
Fig. 22 is a view of the push rod assembly of Fig. 17;
Fig. 23 is a view of the push rod assembly as it is attached to trigger of the applicator of Fig. 17;
Fig. 24 is a view of the guide shaft section of the applicator of Fig. 17;
Fig. 25-27 are views showing construction details of selected portions of the applicator's needle assembly of Fig 17;
Figs. 28-31 are schematic views showing various steps in the use of the applicator in an exemplary tissue repair application; and
Figs. 32-36 are schematic views showing an alternate method in the use of the applicator in an exemplary tissue repair application.

### Detailed Description Of The Preferred Embodiments

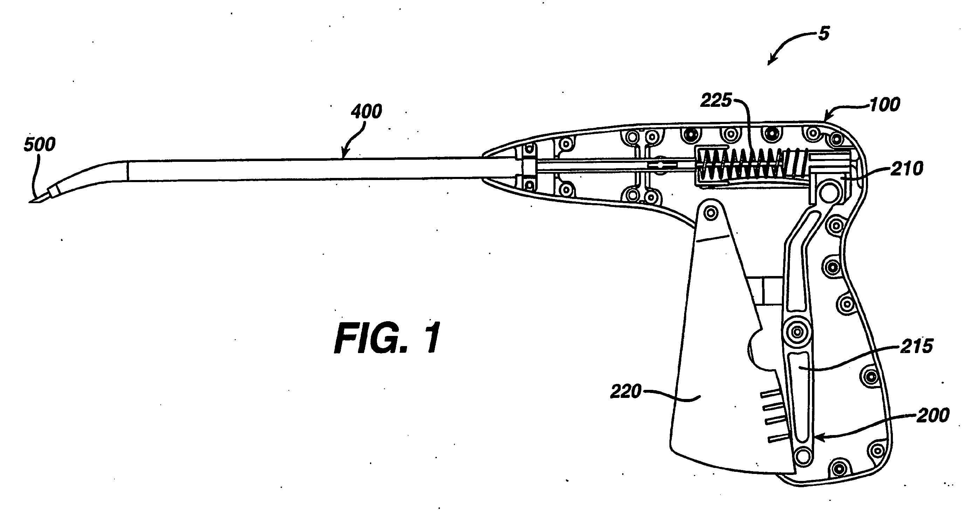

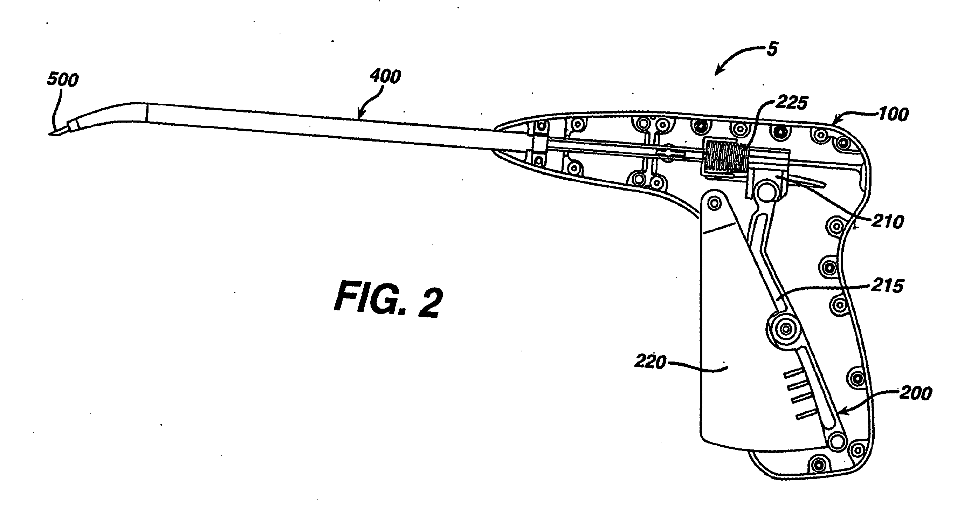

Referring initially to Figs. 1 and 2, there is shown an applicator 5 formed in accordance with the present invention. Applicator 5 has a housing 100, a push rod assembly 200, a guide shaft assembly 400, and a needle assembly 500.

Housing 100 is preferably configured as a pistol grip so as to easily conform to the hand of a user. Housing 100 serves to provide a support structure for the remainder of the elements of the applicator, as will hereinafter be described in further detail. Housing 100 is preferably formed as two mirror halves (only one of which is shown in Fig. 1) so as to simplify manufacture of the applicator, with the two halves being joined during assembly so as to form the complete housing structure.

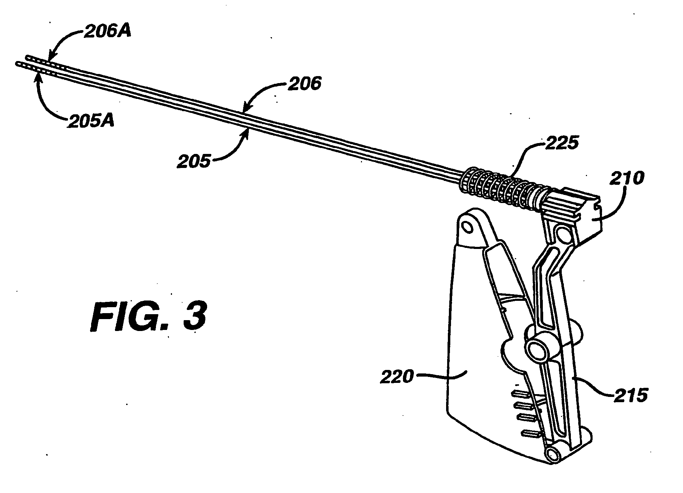

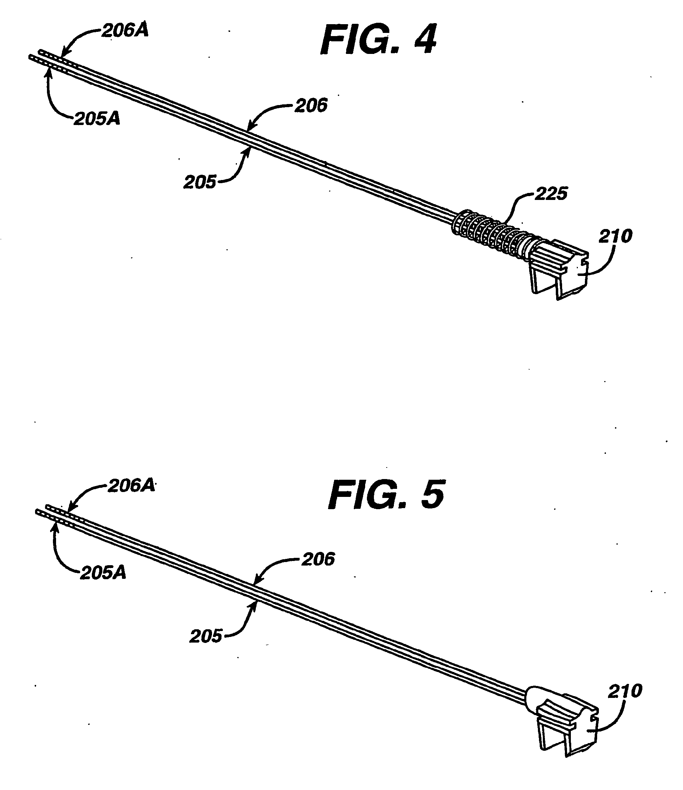

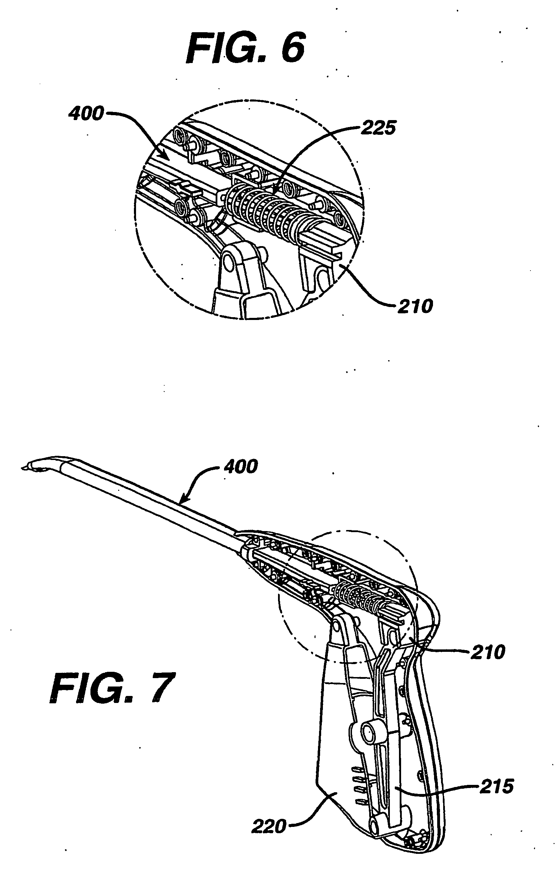

Referring next to Figs. 1, 2 and 3-7, push rod assembly 200 has first and second push rods 205 and 206, respectively, a slide block 210, a pivot lever 215, a trigger or actuator 220, and a compression spring 225. The proximal end of push rods 205 and 206 are preferably solid, and they are secured to slide block 210 so that both push rods 205 and 206 will move in unison with slide block 210. The compression spring 225 is situated around push rods 205 and 206 and connects to slide block 210. The distal end of push rods 205 and 206 contain two flexible but relatively incompressible springs 205A and 206A. The springs facilitate the ability of the push rods 205 and 206 to follow the curvature of needle assembly 500 or the distal end of the guide shaft assembly 400, as will hereinafter be discussed in further detail. Pivot lever 215 and trigger or actuator 220 are pivotally mounted to housing 100. When trigger or actuator 220 is in its forward or first operating position (Fig. 1), slide block 210 and push rod 205 will be in their rearward or second operating positions (Fig. 1), and when trigger 220 is in its rearward position (Fig. 2), slide block 210 and push rod 205 will be in their forward positions (Fig. 2). Compression spring 225 biases trigger 220 into its forward position, and hence slide block 210 and push rod 205 into their rearward positions (Fig. 1).

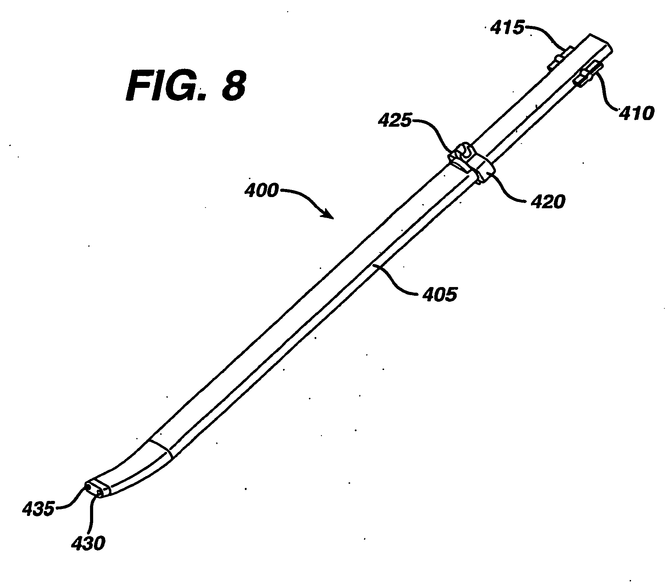

Looking next at Figs. 1-3, 8 and 9, guide shaft section 400, which extends from the housing 100, has a body 405. The proximal end of the body 405 is connected in the handle housing by two sets of tabs located on either side of the shaft. One set is located at the proximal end and is shaped as flat wings 410 and 415, and one set is located mid shaft and shaped as collars 420 and 425. Both sets of tabs have formed mating configurations within both housing halves. These tabs and configurations are designed to secure the guide shaft within the handle housing 100. The inner portion of the guide shaft comprises two channels 430 and 435 that extend the entire length of the guide shaft section 400. The distal ends of these channels are configured to accept two needles 505 and 506, which will be described in more detail below.

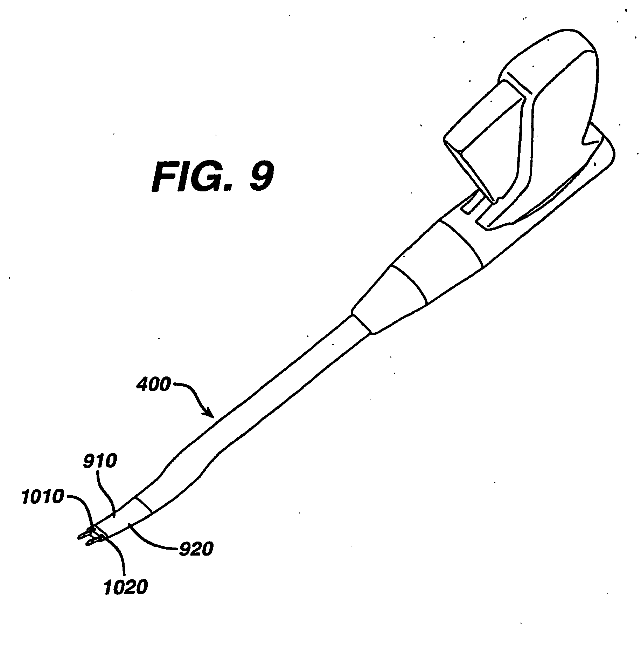

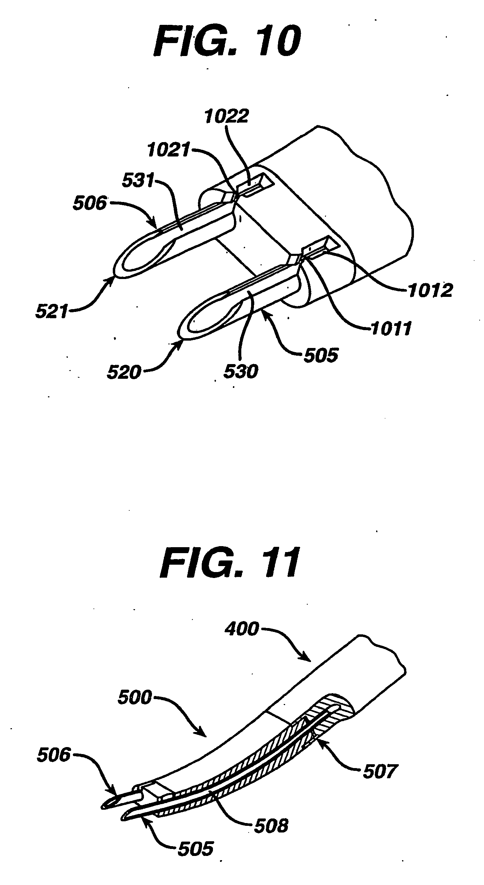

Referring now to Figs. 9, 10, 12 and 13, on the bottom surface of the distal end of guide shaft 400 are first and second fastener receiving and holding receptacles 1010 and 1020. Each receptacle is formed within the body of the shaft and has two separate sections, a gate section 1011 and 1021, and a holding section 1012 and 1022. The gate section is a section of the receptacle at its distal end, which has a narrowed width and beveled surface. The receptacles 1010 and 1020 are sized so that the transverse leg 560 of fastener 515 can slip past the beveled surfaces of gate section 1011 and 1021 and thereafter be releasably captured in the holding sections 1012 and 1022 of the receptacles. The fastener 515 is consequently held in position on needles 505 and 506 during delivery to the surgical site, yet is allowed to separate from the needles under appropriate urging, as will hereinafter be discussed in further detail.

Referring now to Fig 9, on the side surfaces of the distal end of the guide shaft are two protrusions, 910 and 920, which are used to guide the needles and guide shaft into the proper alignment with a fastener cartridge (not shown). The protrusions are generally flat wing like structures. They will mate with corresponding slots in a fastener holding cartridge to ensure proper loading of the fastener into the needles and fastener holding receptacles.

Looking next at Figs. 1, 2 and 10-11, needle assembly 500 has first and second hollow needles 505 and 506 whose proximal ends are attached to the distal end of the shaft assembly by a joint 507. This attachment can be accomplished by gluing, solvent bonding, ultrasonic welding, or injection molding. Needles 505 and 506 can be generally curved along its length or can be straight. The distal ends of needles 505 and 506 terminate in sharp beveled edges 520 and 521. The proximal ends of needles 505 and 506 are sized to be received in channels 508 and 509 (not shown) within the guide shaft. First and second slotted portions 530 and 531, respectively, extend along the length of each needle 505 and 506 and communicate with the interior of each hollow needle. Preferably slotted portions 530 and 531 extend along substantially the entire length of needles 505 and 506; however, if desired, slotted portions 530 and 531 could be formed in only the distal portion of needles 505 and 506, and slotted portions 530 and 531 could be omitted from the proximal portion of the needles.

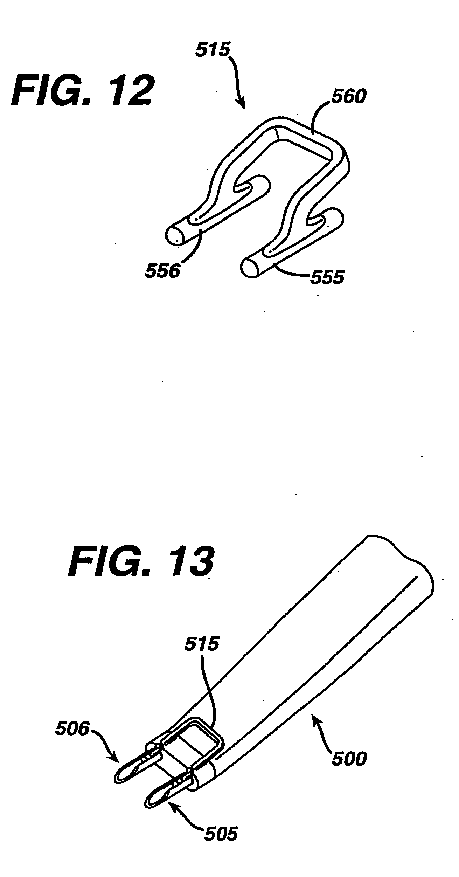

Referring now to Figs. 12 and 13, needles 505 and 506 are sized to receive the legs 555 and 556 of fastener 515. More particularly, fastener 515 is preferably a U-type fastener which has a first leg 555, a second leg 556 generally parallel to the first leg, and a connecting flexible filament 560 (Fig. 12). The fastener 515 may be composed of a non-absorbable material such as polypropylene. Fastener 515 is sized so that its legs 555 and 556 can fit within slotted portions 530 and 531 of needles 505 and 506. When the legs of the fastener are fitted within the slotted portions of the needles, flexible filament 560 of the fastener extends generally perpendicularly to the needles.

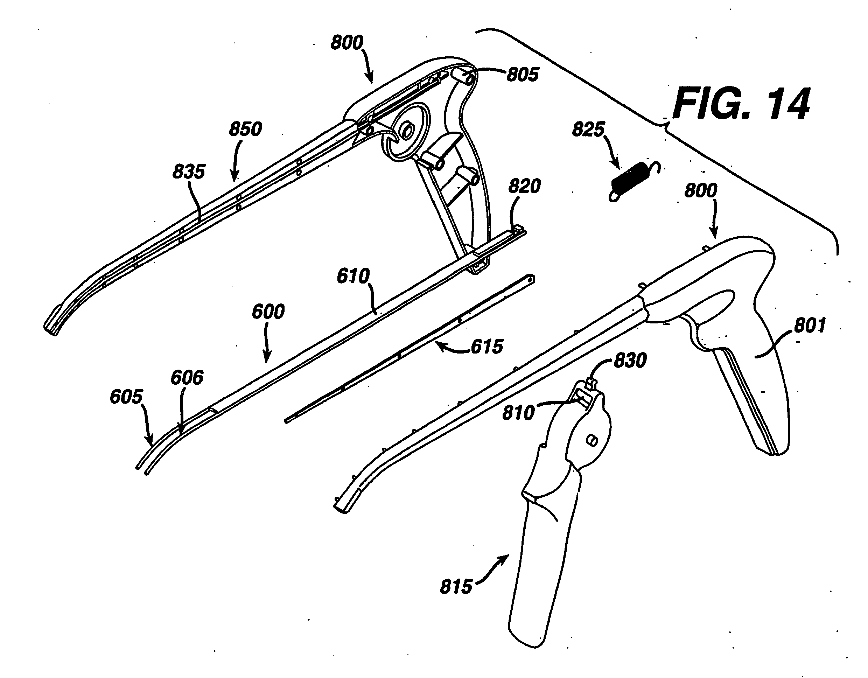

Referring to Figs. 14-16E, there is shown an alternate embodiment of an applicator in accordance with the present invention. Applicator 6 has a housing 800, an actuation assembly 600, and a needle insert 700. Housing 800 has a handle 801 and a gun shaft portion 850, which is one piece. Handle 801 is preferably configured as a pistol grip for ease of use. Housing 800 serves to provide a support structure for the remainder of the elements of the applicator, as will hereinafter be described in further detail. Housing 800 is preferably formed as two halves joined during assembly so as to form the complete housing structure.

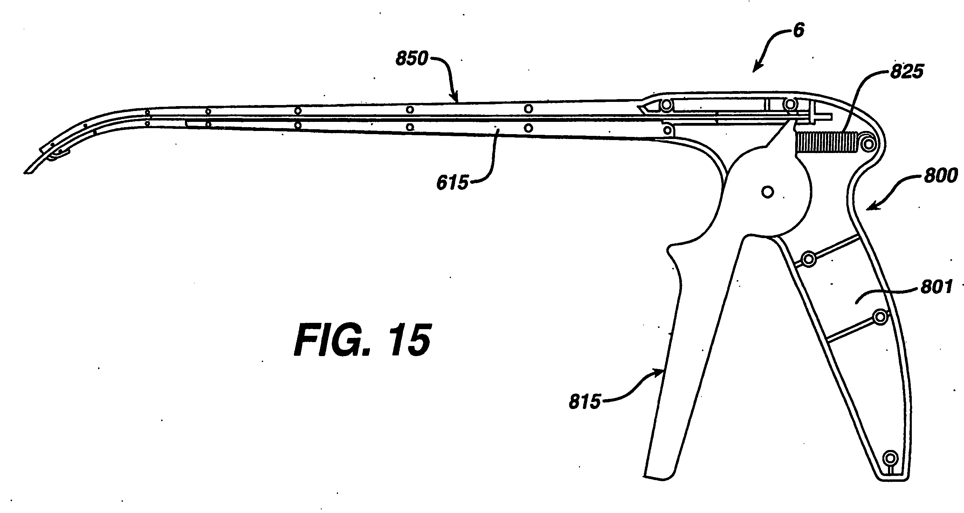

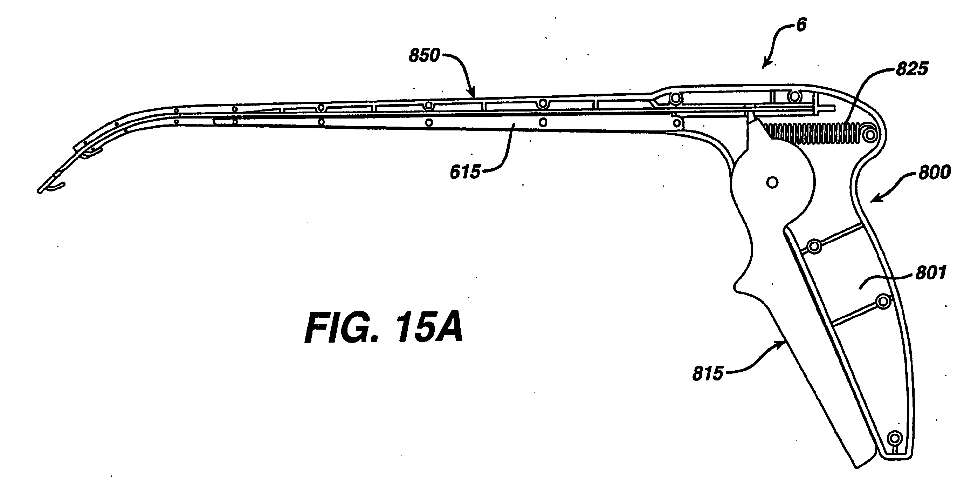

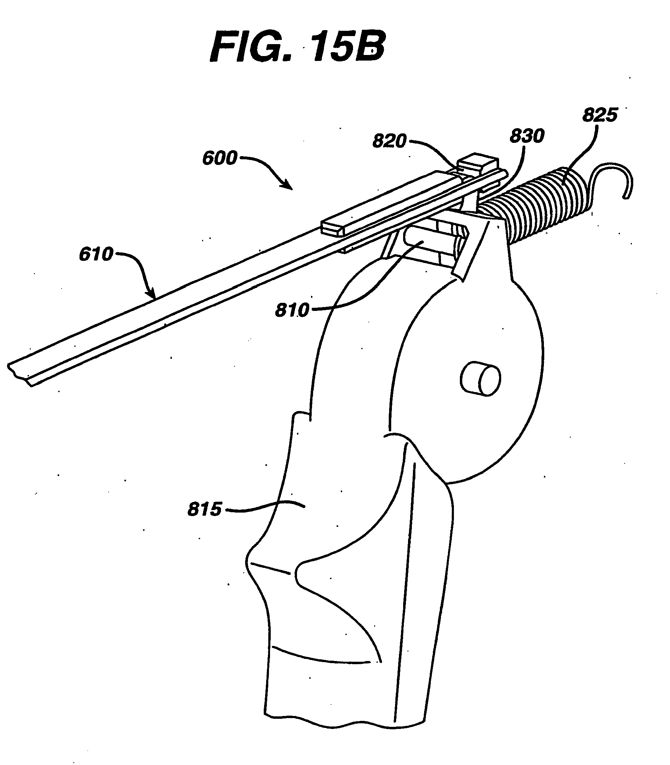

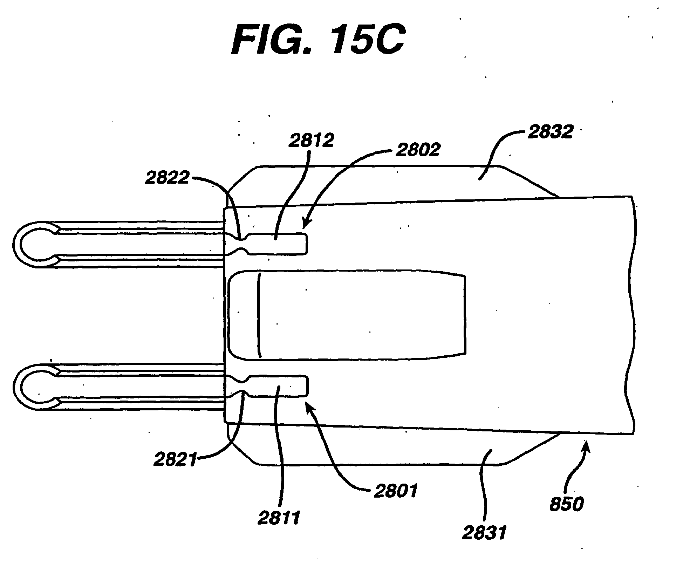

Referring to Figs. 14, 15, 15A and 15B, actuation assembly 600 has a push rod 610, a trigger or actuator 815, and an extension spring 825. The push rod 610 fits snugly into a groove 835 situated on the gun shaft portion 850 of the housing 800, so as to withstand movement forward without buckling. At the proximal end is a square slot 820. This slot is dimensioned to accept a tab 830 situated on the top of trigger 815, so that the push rod 610 will move forward when the trigger 815 is pushed. The extension spring 825 is situated on a cross pin 810 inside the top of the trigger 815 and connects the trigger 815 to the outside diameter of a connecting pin 805 located on the side of the housing 800.

Referring again to Figs 14, 15, 15A and 15B, the distal end of the actuation assembly 600 has two cylindrical rods 605 and 606. These rods are configured to be accepted into the proximal end of the needle insert 700 as will hereinafter be discussed in further detail. Trigger 815 is pivotally mounted to housing 800. When trigger 815 is in its forward or first operating position (Fig. 15), actuation assembly 600 will be in its rearward or position (Figs. 15), and when trigger 815 is in its rearward second operating position (Fig. 15A), actuation assembly 600 will be in its forward position (Fig. 15A). Extension spring 825 biases trigger 815 into its forward position, and hence actuation assembly 600 into its rearward position (Fig. 15).

Referring again to Figures 14 and 15, situated below the actuation assembly 600 on the housing 800 is a reinforcement plate 615. This plate is designed to support the gun shaft portion 850 when the applicator is used during a surgical procedure. As force is applied to the distal end of the applicator and as the actuation assembly 600 is advanced forward, the reinforcement plate supports the actuation assembly 600 and the gun shaft portion 850 preventing buckling of the applicator.

Referring now to Fig. 15C, on the bottom surface of the distal end of gun shaft portion 850 are two fastener receiving and holding receptacles 2801 and 2802. Each receptacle is formed within the body of the shaft and has two separate sections, a gate section 2821 and 2822, and a holding section 2811 and 2812. The gate section is a section of the receptacle at its distal end, which has a narrowed width and beveled surface. The receptacles 2801 and 2802 are sized so that the transverse leg of the fastener can slip past the beveled surfaces of gate sections 2821 and 2822 and thereafter be captured in holding sections 2811 and 2812.

On the side surfaces of the distal end of the gun shaft assembly 850 are two protrusions, 2831 and 2831, which are used to guide the needles and guide shaft into the proper alignment with a fastener cartridge (not shown). The protrusions are generally flat wing like structures. They will mate with corresponding slots in a fastener holding cartridge (not shown) to ensure proper loading of the fastener into the needles and fastener holding receptacles.

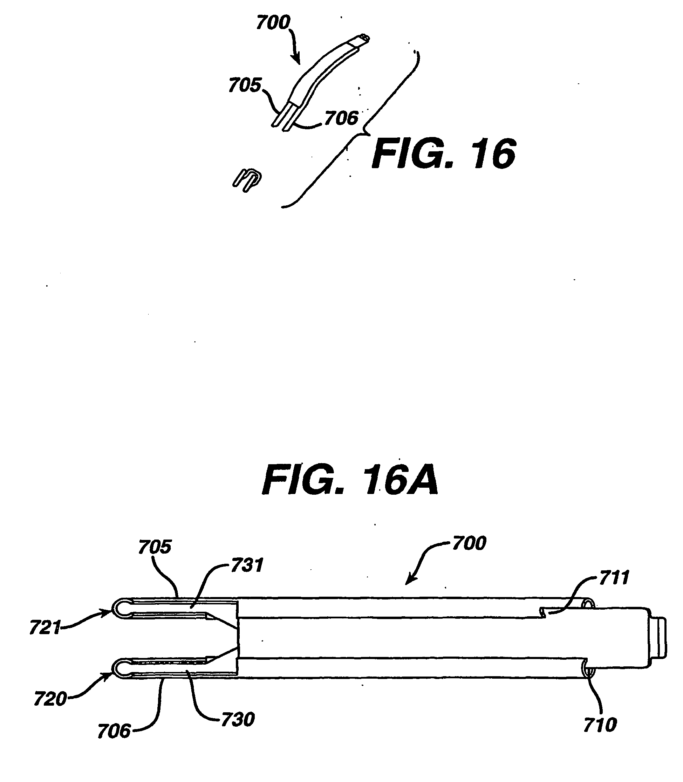

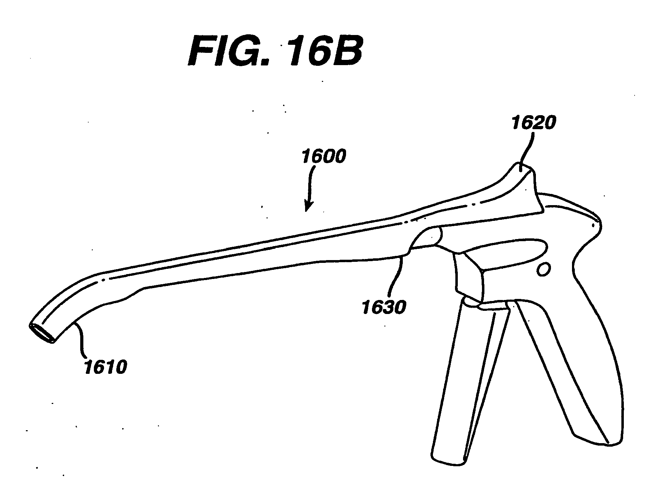

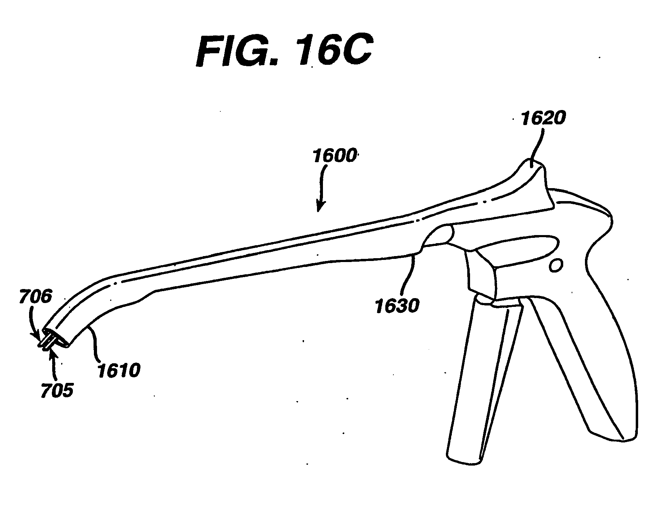

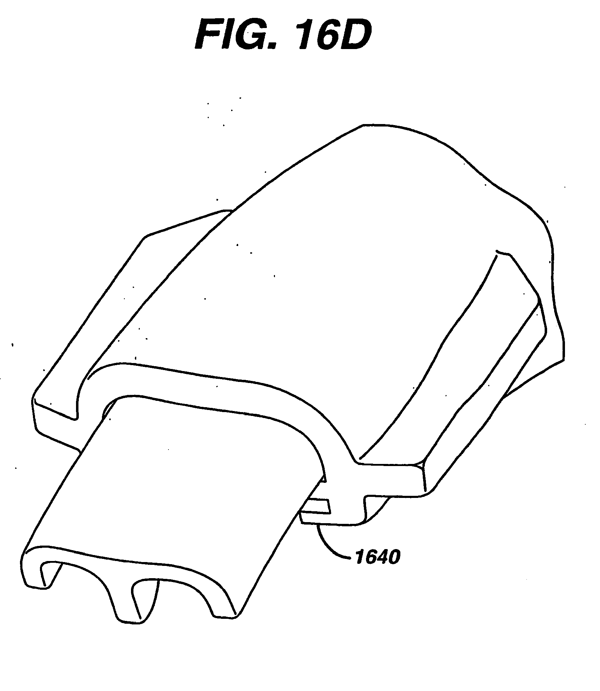

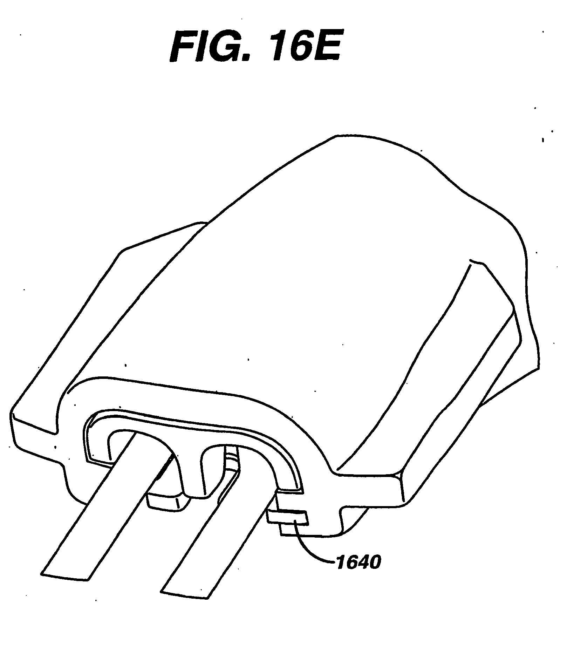

Referring to Figs. 16 and 16A, needle insert 700 is configured as a generally flat rectangular plate. On either side of this rectangular plate are two channels 710 and 711 sized to receive the cylindrical rods 605 and 606 described above. The channel lengths are preferably staggered to facilitate ease of assembly of the cylindrical rods 605 and 606 into the channels 710 and 711. The channels transition to form two hollow needle tips 705 and 706 on the distal end of the needle insert 700. Needle tips 705 and 706 can be generally curved along their length or they can be straight. The distal ends of needle tips 705 and 706 terminate in sharp beveled edges 720 and 721. Two slots 730 and 731 extend along the length of each needle tip 705 and 706 and communicate with the interior of each hollow needle. Preferably slots 730 and 731 extend along substantially the entire length of needle tips 705 and 706; however, if desired, slots 730 and 731 could be formed in only the distal portion of needle tips 705 and 706, and slots 730 and 731 could be omitted from the proximal portion of the needles. Needle tips 705 and 706 are sized so as to releasably capture the legs 555 and 556 of fastener 515 as was described above.

Referring now to Figs. 16B-16C, there is shown an optional shield 1600 for the needles of applicator 6. This may be necessary as the needles may snag or tear tissue as the applicator is inserted into the body, or the user may catch his or her glove on the needles of the applicator. The shield is a tube with a proximal distal ends. The distal end flares into an oval opening 1610. The proximal end terminates in a handle 1620. On the bottom of the proximal end is a curved plate 1630 that can be snapped and locked onto the shaft of the applicator. This can be accomplished by a press fit design or a notch and groove design. The distal end of the applicator is inserted into the proximal end of the shield and the curved plate rides upon and snaps onto the top of the applicator. The shield is sized to receive the applicator and allow the applicator to move axially inside the shield to hide and expose the needle tips. The shield can be fitted to the applicator before the user enters it into the incision, or it can be inserted onto the applicator after the shield has been inserted into the surgical site. Once the assembly is in place at the target site, the shield can be moved rearwardly while pushing the needle tips of the applicator forward to expose the needles. The fastener can then be fired into the target tissue.

Referring now to Figs. 16D and 16E, there is an alternate embodiment to the shield. A second channel 1640 is created in the applicator shaft to hold a drive pin located on the shield, which is located on the distal end of the applicator to cover the needle assembly. This pin connects the shield to a spring and actuator assembly in the handle of the applicator. The user is able to pull the shield back into the applicator shaft by depressing a button, or other type of mechanism on the applicator's handle. The shield may be biased forward so that it is always covering the needles (Fig 16D). After the applicator is inserted into the incision and placed in the correct area, the user would depress a button that would pull the shield back into the applicator's shaft exposing the needles (Fig. 16E) and allowing the user to press the needles of the applicator into the site. Furthermore, the internal shield could be integrated with a trigger lock in such a way that the trigger can only be fired when the shield is in the retracted position.

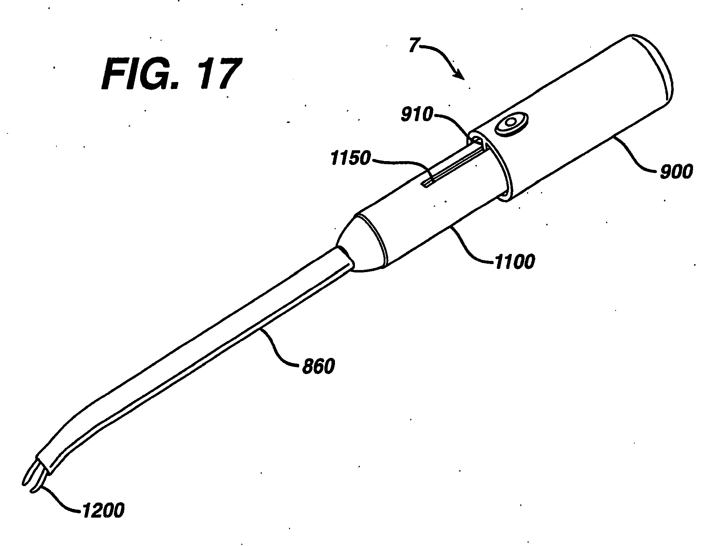

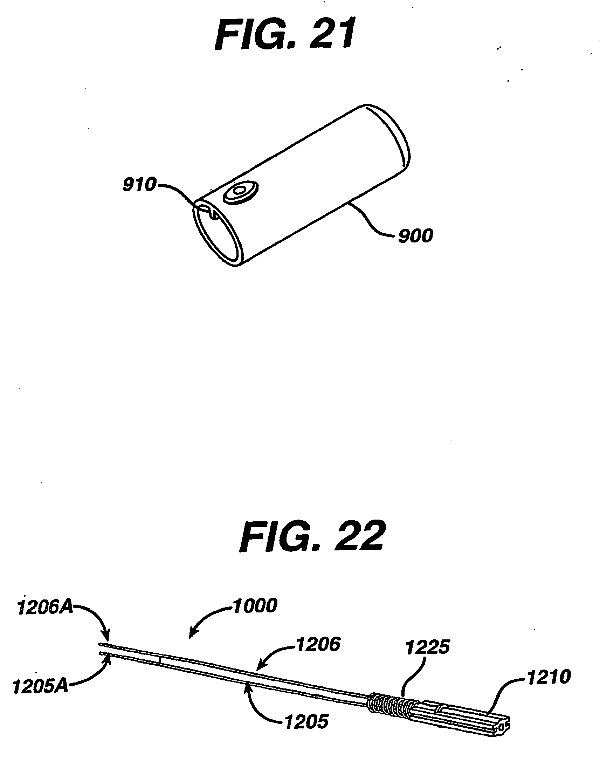

Referring now to Figs. 17-22, there is shown yet another embodiment of an applicator in accordance with the present invention. Applicator 7 has a trigger or actuator 900, a guide shaft assembly 850, a push rod assembly 1000, a housing 1100 and a needle assembly 1200. The trigger 900 is configured as a hollow tube that is situated over the housing 1100. It is generally cylindrical in shape, and its function is to advance the push rod assembly 1000 forward or backward, as will be described in more detail below. Trigger 900 is attached to the housing by a key 910 positioned on the inside diameter of the trigger that slides in an elongated slot 1150, which restricts lateral movement of the trigger.

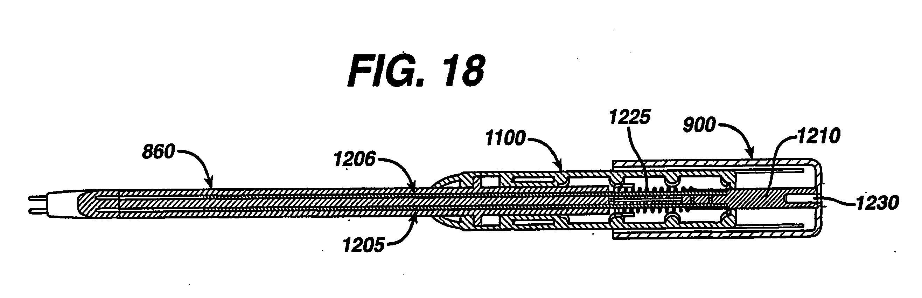

Another function of housing 1100 is to provide a support structure for the remainder of the elements of the applicator, as will hereinafter be described in further detail. Housing 1100 is preferably formed as two mirror halves (only one of which is shown in Fig. 18) so as to simplify manufacture of the applicator, with the two halves being joined during assembly so as to form the complete housing structure.

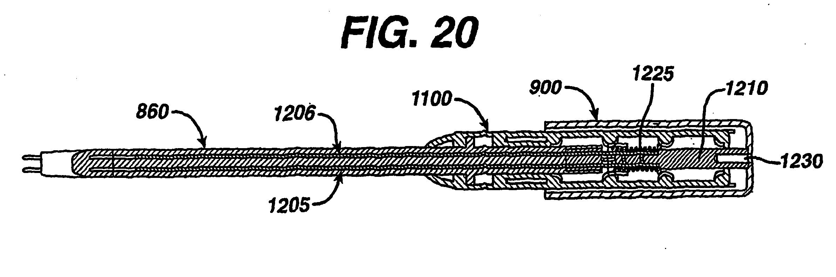

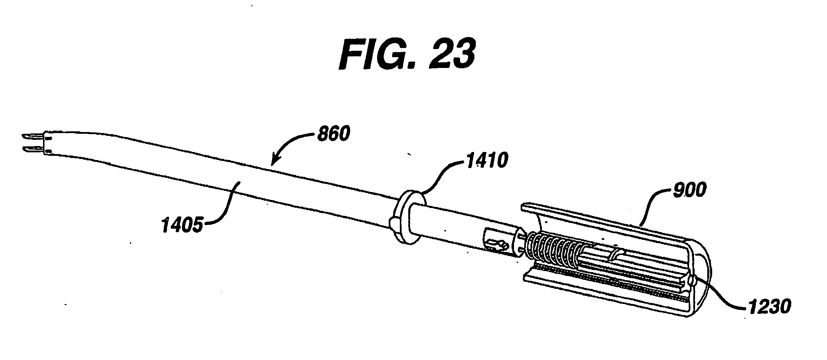

Referring now to Figs. 18-20, 22 and 23, push rod assembly 1000 has two push rods 1205 and 1206, a slide block 1210, and a compression spring 1225. The proximal end of push rods 1205 and 1206 are preferably solid, and they are secured to slide block 1210 so that both push rods 1205 and 1206 will move in unison with slide block 1210 when the trigger is advanced forward. The slide block is secured to the trigger by a screw 1230. The compression spring 1225 is situated around push rods 1205 and 1206 and connects to slide block 1210. The distal end of push rods 1205 and 1206 contain two flexible but relatively incompressible springs 1205A and 1206A. This enables the push rods 1205 and 1206 to follow the curvature of needle assembly 1200 as will hereinafter be discussed in further detail. Trigger is coaxially mounted to housing 1100, whereby (i) when trigger 900 is in its resting or first operating position (Fig. 18), slide block 1210 and push rods 1205 and 1206 will be in their rearward positions (Fig. 18), and (ii) when trigger 900 is in its forward or second operating position (Fig. 20), slide block 1210 and push rods 1205 and 1206 will be in their forward positions (Fig. 20). Compression spring 1225 biases trigger 900 into its resting position, and hence slide block 1210 and push rods 1205 and 1206 into their rearward positions (Fig. 20).

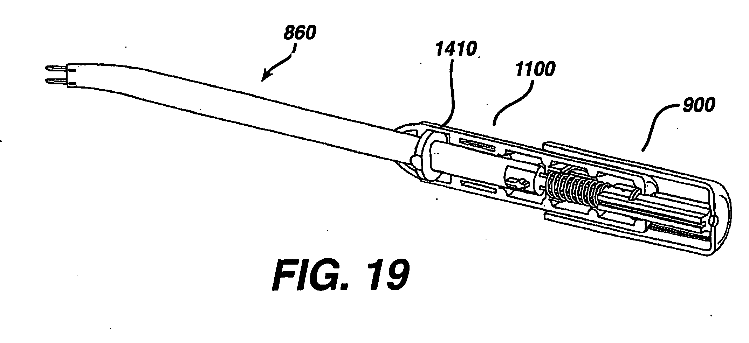

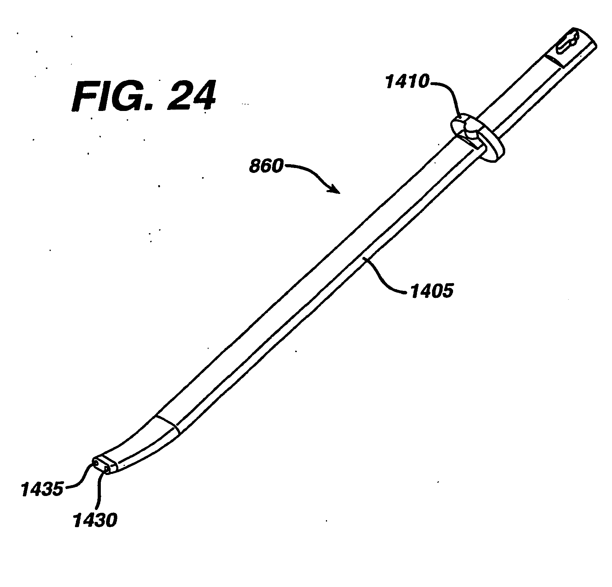

Referring to Figs. 19, and 23-25, guide shaft section 860, which extends from the housing 1100, has a body 1405 and proximal and distal ends. The proximal end is connected in the housing by a flange 1410 located on the proximal end of the shaft. This is designed to secure the guide shaft within the housing. The inner portion of the guide shaft comprises two channels 1430 and 1435 that extend the entire length of the guide shaft section 860. The distal ends of these channels receive two needles 2505 and 2506, which will be described in more detail below.

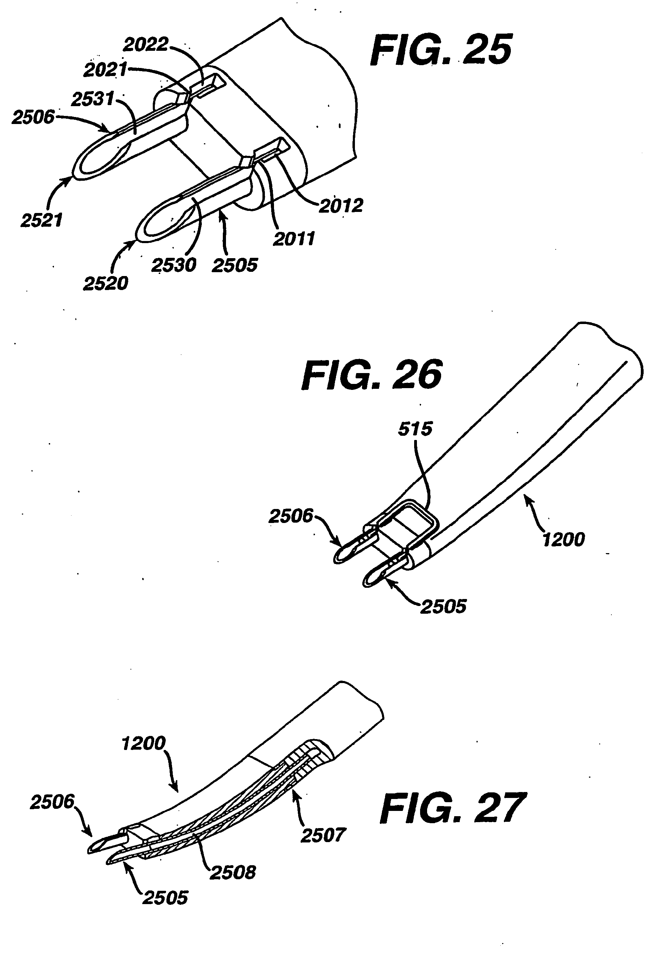

Referring now to Figs. 25-27, needle assembly 1200 has two hollow needles 2505 and 2506 whose proximal ends are attached to the distal end of the shaft assembly by a joint 2507. This attachment can be accomplished by gluing, solvent bonding, ultrasonic welding, or injection molding. Needles 2505 and 2506 can be generally curved along its length or can be straight. The distal ends of needles 2505 and 2506 terminate in sharp beveled edges 2520 and 2521. The proximal ends of needles 2505 and 2506 are sized to be received within channels 2508 and 2509. First and second slotted portions 2530 and 2531, respectively, extend along the length of each needle 2505 and 2506 and communicate with the interior of each hollow needle. Preferably slotted portions 2530 and 2531 extend along substantially the entire length of needles 2505 and 2506; however, if desired, slotted portions 2530 and 2531 could be formed in only the distal portion of needles 2505 and 2506, and slotted portions 2530 and 2531 could be omitted from the proximal portion of the needles. Needles 2505 and 2506 are sized to capture the legs 555 and 556 of fastener 515 as was described in detail previously.

By way of example but not limitation, the operation of applicator 5 will be discussed in the context of using fastener 515 to repair a pelvic organ prolapse, specifically a cystocele, although many other applications of the present invention will be readily apparent to those skilled in the art.

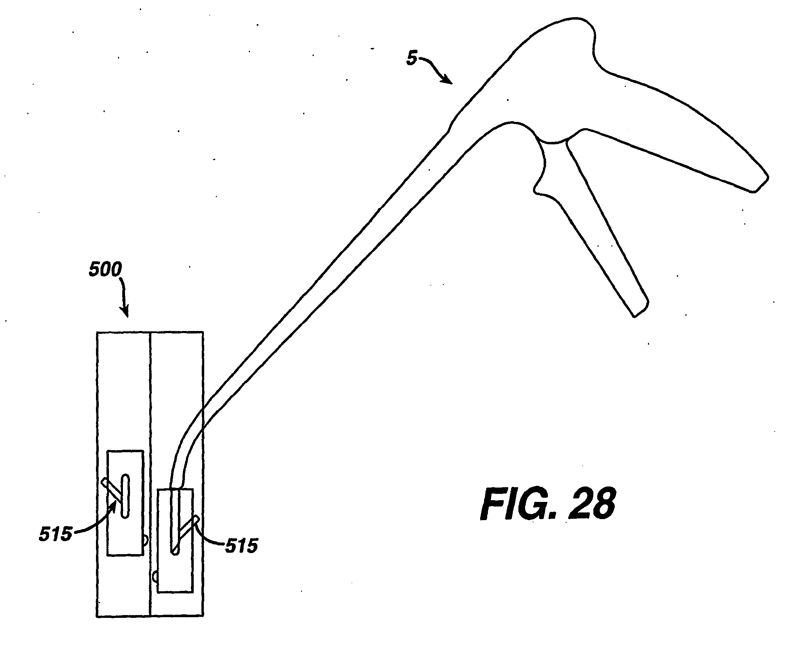

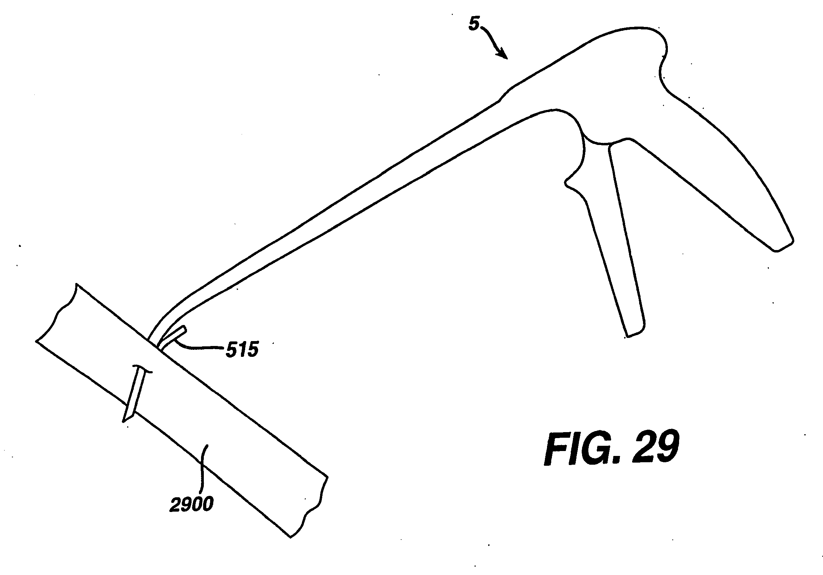

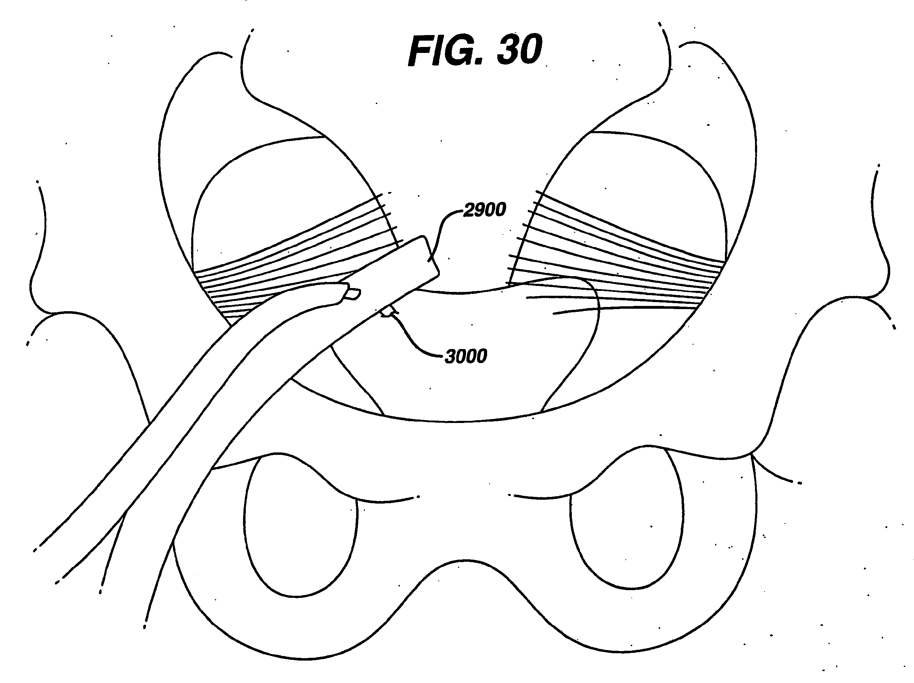

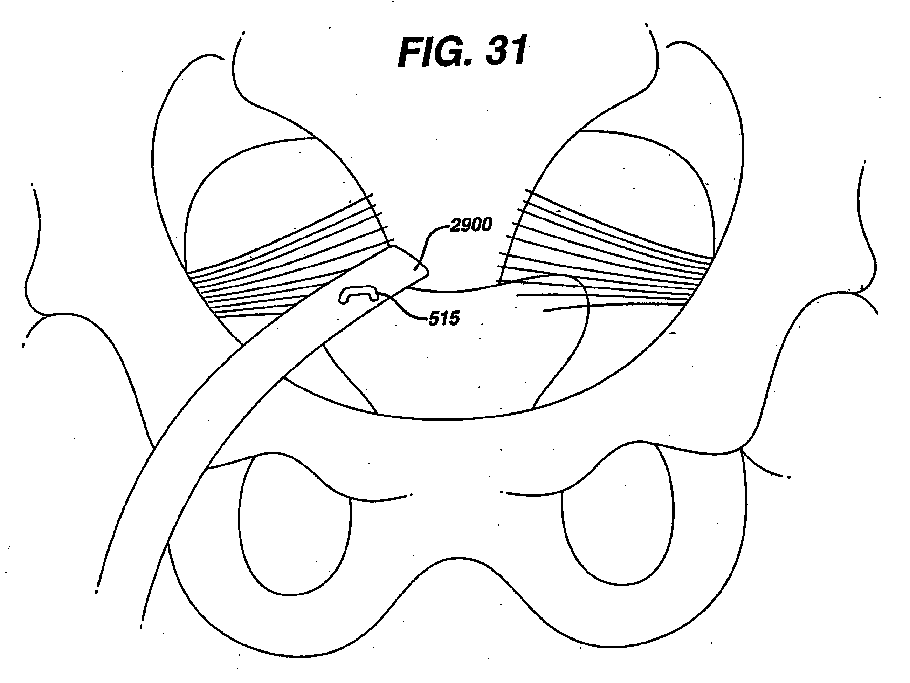

Referring now to Figs. 28-31, the user prepares applicator 5 to pick up fastener 515. More particularly, the user picks up applicator 5, which is normally in the configuration shown in Fig. 1 (i.e., with trigger or actuator 220 out). The user then readies applicator 5 to receive fastener 515, by passing the wings on the distal end of the applicator into the grooves on a cartridge 5000, which may hold at least one fastener. The distal end of the applicator is advanced so that the legs of the fastener are slid into the slots of the needle assembly and an audible click is heard. At this point fastener 515 is withdrawn from the cartridge using applicator 5.

Once the applicator and fastener are ready for use, the user makes a vaginal incision in the anterior wall of the vagina. This is done to gain access to the muscles, ligaments, and other tissue structures of the pelvic floor. Next, the distal end of the applicator is secured to a mesh implant 2900 by pushing the needle assembly into the weave of the mesh. The distal end of the applicator 5 with mesh attached is advanced through the vaginal incision and aligned with the target attachment site 3000. The applicator and mesh 2900 are then placed on the target attachment site 3000 and the needles are advanced to penetrate the tissue. Then, while keeping pressure on the handle of applicator 5, the user depresses trigger 220 (Fig. 2) so as to cause push rods 205 and 206 to advance. The distal ends of push rods 205 and 206 engage legs of fastener 515 and eject it out the distal end of needle assembly. In this respect it is to be appreciated that the flexible construction of the distal ends 205A and 206A of push rods 205 and 206 permit the push rods to follow the curvature of needles 505 and 506 as the fastener is ejected from the needles. As the fastener's legs penetrate the target site it forms a U shape around the mesh and the target site. This procedure is then repeated as many times as needed to secure the mesh to the target tissue.

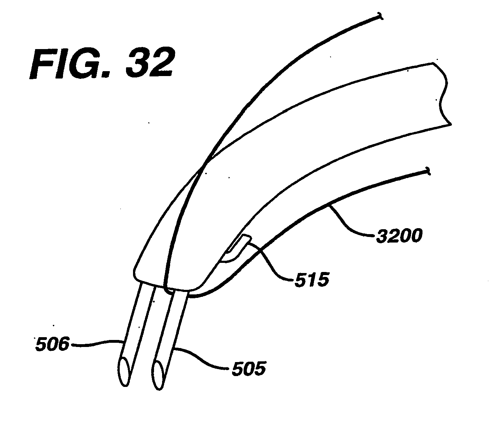

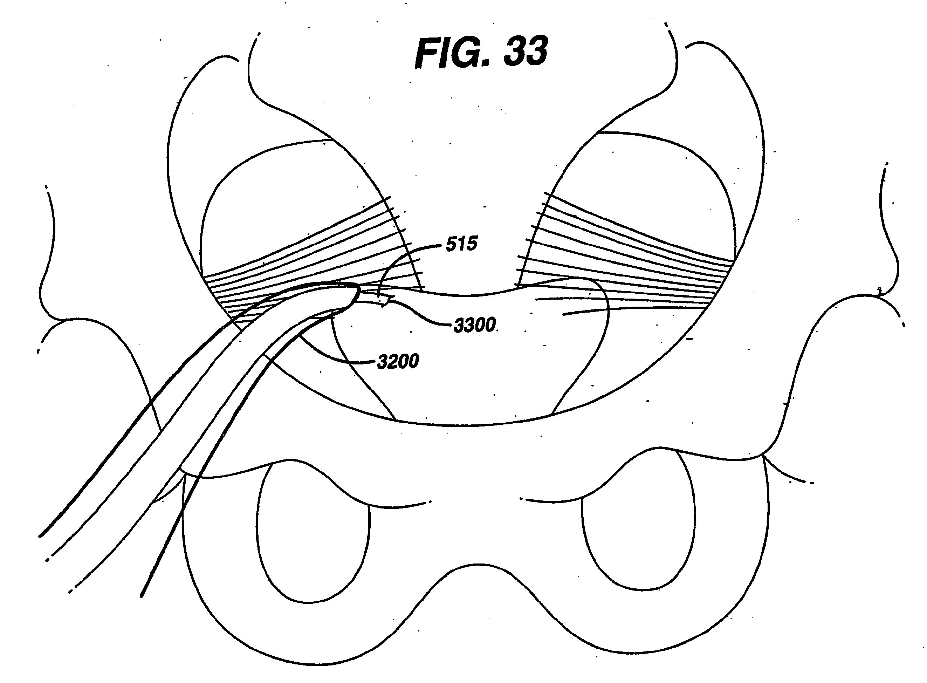

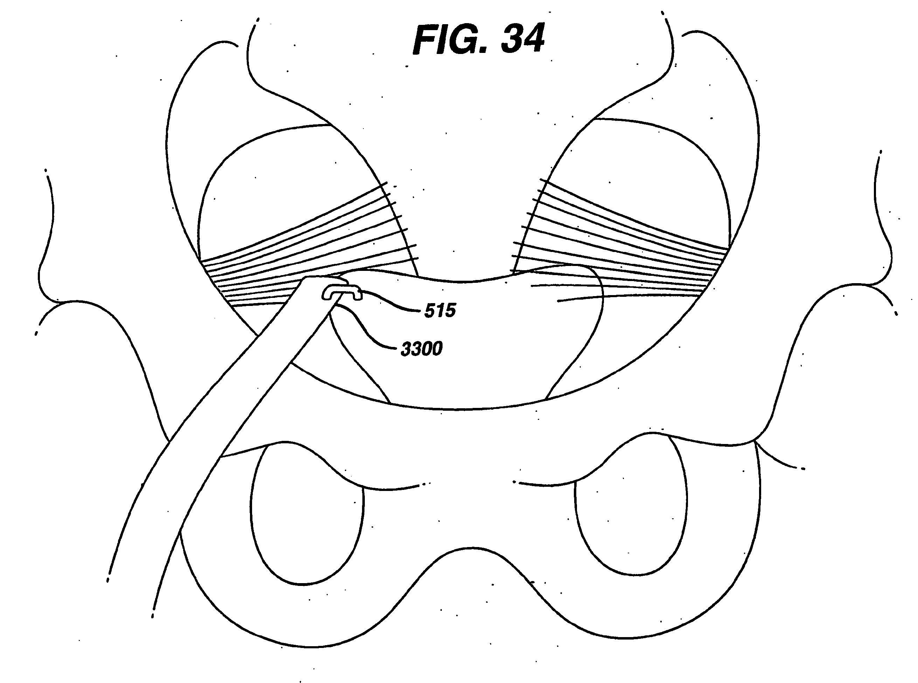

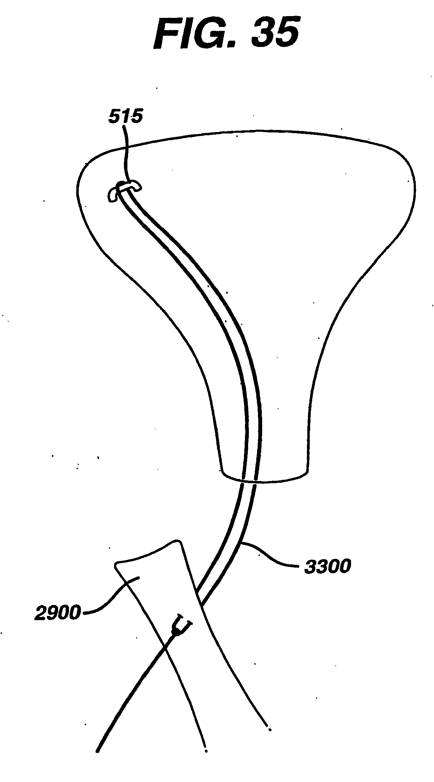

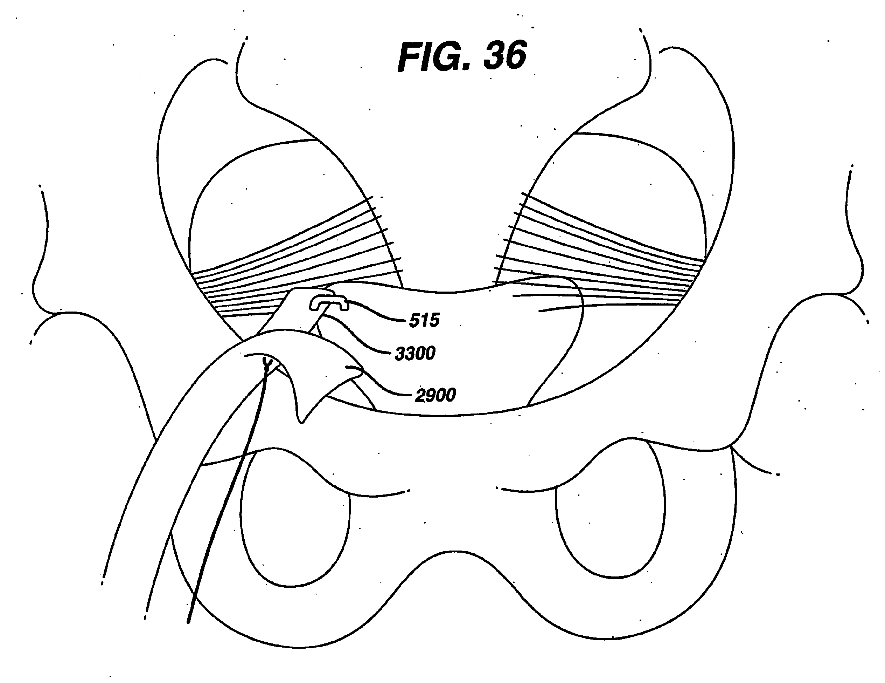

An alternate embodiment of performing pelvic organ prolapse repair using the applicator of the present invention will now be described. Referring now to Figs. 32-36, the user prepares applicator 5 to pick up fastener 515 as described above. Once the applicator and fastener are ready for use, the user makes a vaginal incision in the anterior wall of the vagina as described above. Next, a length of suture 3200 is placed between the legs of the fastener and the two needles 505 and 506 of the needle assembly on the applicator. The distal end of the applicator 5 with suture positioned and fastener in place is advanced through the vaginal incision and aligned with the target attachment site. The applicator, fastener and suture are then placed on the target attachment site 3300 with the needles penetrating the target tissue. Then, while keeping pressure on the handle of applicator 5, the user depresses trigger 220 (Fig. 2) so as to cause push rods 205 and 206 to advance. The distal ends of push rods 205 and 206 engage legs of fastener 515 and eject it out the distal end of needle assembly. In this respect it is to be appreciated that the flexible construction of the distal ends 205A and 206A of push rods 205 and 206 permit the push rods to follow the curvature of needles 505 and 506 as the fastener is ejected from the needles. As the legs of the fastener penetrate the target site, it captures both the suture and the target tissue site and forms a U shape around the suture and the target site.

Next, the loose ends of the suture 3300 are then positioned outside the vagina and the user passes these loose ends through a mesh implant 2900, and then crosses the ends of the suture one over the other. The suture ends are then cinched down with the mesh to the target tissue site and the fastener. The suture is then tied using conventional suture knot tying techniques. In this way, the suture adds an adjustability feature to the mesh placement, as the mesh can then be tightened or loosened as needed. This procedure is then repeated as many times as needed to secure the mesh to the target tissue.

It is, of course, possible to modify the preferred embodiments of the applicator without departing from the scope of the present claims. For example, it is possible to use the applicator of the present invention in a procedure other than the one described above, e.g., one might use the applicator to attach two pieces of tissue in the chest, abdomen, heart, or pelvic cavity. One might form needle assembly 500 so that it incorporates straight needles 505A and 506A rather than the curved needles 505 and 506 discussed above. If the needles were straight, then the distal ends 205A and 206A of push rods 205 and 206 could be rigid instead of flexible, since it would not need to traverse a curved arc as in the case where a curved needle is used. Alternatively, the distal ends of the push rods could be composed of a flexible plastic, or compliant metal with superelasticity such as Nitinol shape-memory alloy. In still yet another embodiment, it is possible to use applicator of the present invention with other double-legged fasteners rather than with the U-type fastener 515 described above.

Still other variations obvious to a person skilled in the art are considered to be within the scope of the present invention as shown by the appended claims.

## Claims

1. An applicator (5) for deploying a fastener (515) into tissue, said applicator (5) having a distal end, the fastener (515) including first and second legs (555, 556) generally parallel to each other and connected by a transverse leg (560), said applicator (5) comprising:
a first elongated hollow needle member (505) having a first distal end for penetrating tissue and a first slotted portion at the first distal end for releasably retaining said first leg (555) of the fastener (515) in said first slotted portion of said first elongated hollow needle member (505);
a second elongated hollow needle member (506) generally parallel to said first elongated hollow needle member (505), said second elongated hollow needle member (506) having a second distal end for penetrating tissue and a second slotted portion at the second distal end for releasably retaining said second leg (556) of the fastener (515) in said second slotted portion of said second elongated hollow needle member (506);
first and second push rods (205, 206) adapted to move axially from rearward to forward positions within said first and second elongated hollow needle members (505, 506); and
an actuator (200) engageable with said first and second push rods (205, 206) for moving said first and second push rods (205, 206) in said first and second elongated hollow needle members (505, 506) from the rearward to the forward positions so as to eject the first and second legs (555, 556) of the fastener (515) from said first and second slotted portions of said first and second elongated hollow needle members (505, 506);
said applicator (5) further comprising first and second receptacles (1010, 1020, 2801, 2802) at the applicator distal end, said receptacles (1010, 1020, 2801, 2802) being capable of receiving said transverse leg (560) and thereby releasably fixing the legs (555, 556) of said fastener (515) within the slotted portions of the needle members (505, 506).

2. An applicator (5) according to claim 1 wherein said first and second elongated hollow needle members (505, 506) are curved.

3. An applicator (5) according to claim 1 wherein said actuator (200) further comprises a spring-biased trigger coupled to said first and second push rods (205, 206).

4. An applicator (5) according to claim 1 wherein said distal end portion of said push rods (205, 206) each comprise a flexible spring (205A, 206A) which is substantially incompressible.

5. An applicator (5) according to Claim 1 further comprising a shield (1600) which at least partially covers said first and second elongated hollow needle members (505, 506).

6. An applicator (5) according to Claim 1 wherein the applicator (5) has an applicator distal end, and the applicator (5) further comprises a wing (910, 920) on the applicator distal end.

## Patentansprüche

1. Applikator (5) zur Ablage eines Befestigungselements (515) in Geweben, wobei der Applikator (5) ein distales Ende aufweist, das Befestigungselement (515) erste und zweite Schenkel (555, 556) enthält, die sich allgemein parallel zueinander erstrecken und durch einen Querschenkel (560) verbunden sind, wobei der Applikator (5) aufweist:
ein erstes längliches Hohlnadelelement (505) mit einem ersten distalen Ende zum Penetrieren von Gewebe und einem ersten geschlitzten Abschnitt an dem ersten distalen Ende zum lösbaren Halten des ersten Schenkels (555) des Befestigungselements (515) in dem ersten geschlitzten Abschnitt des ersten länglichen Hohlnadelelements (505);
ein zweites längliches Hohlnadelelement (506), das sich allgemein parallel zum ersten länglichen Hohlnadelelement (505) erstreckt, wobei das zweite längliche Hohlnadelelement (506) ein zweites distales Ende zum Penetrieren von Gewebe und einen zweiten geschlitzten Abschnitt an dem zweiten distalen Ende zum lösbaren Halten des zweiten Schenkels (556) des Befestigungselements (515) in dem zweiten geschlitzten Abschnitt des zweiten länglichen Hohlnadelelements (506) aufweist;
erste und zweite Schubstangen (205, 206), die fähig sind, sich von hinteren zu vorderen Positionen in den ersten und zweiten länglichen Hohlnadelelementen (505, 506) axial zu bewegen; und
einen Aktuator (200), der mit den ersten und zweiten Schubstangen (205, 206) zum Bewegen der ersten und zweiten Schubstangen (205, 206) in den ersten und zweiten länglichen Hohlnadelelementen (505, 506) aus den hinteren zu den vorderen Positionen in Eingriff bringbar ist, um die ersten und zweiten Schenkel (555, 556) des Befestigungselements (515) aus den ersten und zweiten geschlitzten Abschnitten der ersten und zweiten länglichen Hohlnadelelemente (505, 506) auszustoßen;
wobei der Applikator (5) ferner erste und zweite Aufnahmen (1010, 1020, 2801, 2802) an dem distalen Ende des Applikators aufweist und die Aufnahmen (1010, 1020, 2801, 2802) zur Aufnahme des Querschenkels (560) und **dadurch** zum lösbaren Befestigen der Schenkel (555, 556) des Befestigungselements (515) in den geschlitzten Abschnitten der Nadelelemente (505, 506) fähig sind.

2. Applikator (5) nach Anspruch 1, **dadurch gekennzeichnet, dass** die ersten und zweiten länglichen Hohlnadelelemente (505, 506) gekrümmt sind.

3. Applikator (5) nach Anspruch 1, **dadurch gekennzeichnet, dass** der Aktuator (200) ferner einen federnd vorgespannten Abzug aufweist, der mit den ersten und zweiten Schubstangen (205, 206) gekoppelt ist.

4. Applikator (5) nach Anspruch 1, **dadurch gekennzeichnet, dass** der distale Endabschnitt der Schubstangen (205, 206) jeweils eine flexible Feder (205A, 206A) aufweist, die im wesentlichen nicht komprimierbar ist.

5. Applikator (5) nach Anspruch 1, ferner umfassend eine Abschirmung (1600), die die ersten und zweiten länglichen Hohlnadelelemente (505, 506) wenigstens teilweise bedeckt.

6. Applikator (5) nach Anspruch 1, **dadurch gekennzeichnet, dass** der Applikator (5) ein distales Applikatorende aufweist und der Applikator (5) ferner einen Flügel (910, 920) an dem distalen Applikatorende aufweist.

## Revendications

1. Applicateur (5) pour déployer une agrafe (515) dans un tissu, ledit applicateur (5) ayant une extrémité distale, l'agrafe (515) comprenant une première et une seconde pattes (555, 556) généralement parallèles l'une à l'autre, et reliées par une patte transversale (560), ledit applicateur (5) comprenant :
• un premier élément à aiguille creuse allongé (505) ayant une première extrémité distale, destinée à pénétrer le tissu et une première portion fendue, au niveau de la première extrémité distale pour retenir de manière amovible ladite première patte (555) de l'agrafe (515) dans ladite première portion fendue dudit premier élément à aiguille creuse allongé (505) ;
• un second élément à aiguille creuse allongé (506) généralement parallèle audit premier élément à aiguille creuse allongé (505), ledit second élément à aiguille creuse allongé (506) ayant une seconde extrémité distale, destinée à pénétrer le tissu et une seconde portion fendue, au niveau de la seconde extrémité distale, afin de retenir de manière amovible ladite seconde patte (556) de l'agrafe (515) dans ladite seconde portion fendue dudit second élément à aiguille creuse allongé (506) ;
• une première et une seconde tiges de poussoir (205, 206) adaptées pour se déplacer axialement des positions arrière à avant dans lesdits premier et second éléments à aiguille creuse allongés (505, 506) ; et
• un système d'actionnement (200) pouvant être mis en prise avec lesdites première et seconde tiges de poussoir (205, 206), afin de déplacer lesdites première et seconde tiges de poussoir (205, 206) dans lesdits premier et second éléments à aiguille creuse allongés (505, 506) des positions arrière vers l'avant, de façon à éjecter la première et la seconde pattes (555, 556) de l'agrafe (515) desdites première et seconde portions fendues desdits premier et second éléments à aiguille creuse allongés (505, 506) ;
ledit applicateur (5) comprenant en outre un premier et un second réceptacles (1010, 1020, 2801, 2802) au niveau de l'extrémité distale de l'applicateur, lesdits réceptacles (1010, 1020, 2801, 2802) étant capables de recevoir ladite patte transversale (560) et ainsi de fixer de manière amovible les pattes (555, 556) de ladite agrafe (515) dans les portions fendues des éléments à aiguille (505, 506).

2. Applicateur (5) selon la revendication 1, dans lequel lesdits premier et second éléments à aiguille creuse allongés (505, 506) sont incurvés.

3. Applicateur (5) selon la revendication 1, dans lequel ledit système d'actionnement (200) comprend en outre un déclencheur chargé par ressort raccordé auxdites première et seconde tiges de poussoirs (205, 206).

4. Applicateur (5) selon la revendication 1, dans lequel lesdites portions d'extrémité distale desdites tiges de poussée (205, 206) comprennent chacune un ressort flexible (205A, 206A) qui est sensiblement incompressible.

5. Applicateur (5) selon la revendication 1, comprenant en outre un écran (1600) qui couvre au moins partiellement lesdits premier et second éléments à aiguille creuse (505, 506).

6. Applicateur (5) selon la revendication 1, dans lequel l'applicateur (5) a une extrémité distale d'applicateur, et l'applicateur (5) comprend une ailette (910, 920) sur l'extrémité distale dudit applicateur.
